# EUROPEAN PATENT SPECIFICATION

(11) **EP 2 029 067 B1**
(45) Date of publication and mention of the grant of the patent: **18.07.2012**
(21) Application number: 07747284.3
(22) Date of filing: 17.04.2007
(51) Int. Cl.: A61F 5/00

(54) **A KNEE BRACE AS WELL AS A HINGE MEMBER FOR SUCH A BRACE**
KNIESCHIENE UND SCHARNIERELEMENT FÜR DIE SCHIENE
ORTHÈSE DE GENOU ET ÉLÉMENT DE CHARNIÈRE POUR CELUI-CI

(30) Priority: 16.06.2006 NL 1032022
(43) Date of publication of application: 04.03.2009
(73) Proprietor: Nea International B.V., ZN 6199 Maastricht Airport (NL)
(72) Inventor: VOSKUILEN, Agnes Theodora Maria, 6229 TB Maastricht (NL); KOESLAG, Jeroen Hendrik Johan, 6211 EL Maastricht (NL)
(74) Representative: Algemeen Octrooi- en Merkenbureau
(86) International application number: PCT/NL2007/000102
(87) International publication number: WO 2007/145504

(56) References cited:
- EP-A- 0 941 722
- DE-A1- 10 147 243
- US-A- 4 781 180
- US-A- 4 873 967
- US-A- 5 460 599
- US-A- 5 857 989
- US-A1- 2003 204 156

## Description

The invention relates to a knee brace which can be detachably attached to a leg comprising an upper leg portion, a knee portion and a lower leg portion comprising a shin portion, which leg can pivot from an extended position to a flexed position and vice versa, which knee brace at least comprises at least one upper member to be attached to the upper leg portion, at least one lower member to be connected to the lower leg portion, at least one hinge member, which is pivotally connected to the upper member by a first pivot pin and which is pivotally connected to the lower member by a second pivot pin, and means for exerting an anterior-posterior shearing force on the lower leg relative to the upper leg upon pivoting of the leg from the flexed position to the extended position.

The invention also relates to a hinge member suitable for use with such a knee brace.

With such a knee brace, which is known from US patent US-A-4,955,369, two parallel upper members positioned on either side of the leg are strapped to the upper leg portion. In a similar fashion, two lower members extending parallel to each other are strapped to a lower leg portion. Each upper member is connected to a lower member positioned therebelow by means of a hinge member. The knee brace further comprises a guide member, which is connected to the lower member by a pivot pin and which is provided with an elongated slot. In said elongated slot, pins connected to the upper member and to the hinge member are located, which pins are capable of sliding as well as rotary movement in said slot.

Upon pivoting of a leg provided with said knee brace from a flexed position to an extended position, a relative anterior-posterior shift takes place between the upper and lower members, as a result of which an anterior-posterior shearing force is exerted on the lower leg relative to the upper leg.

The exertion of such a shearing force is desirable in the case of persons whose anterior cruciate ligament no longer functions. Without such an external anterior-posterior shearing force being exerted on the lower leg relative to the upper leg, the absence of said anterior cruciate ligament would lead to an undesirable shift in anterior direction of the lower leg relative to the upper leg, so that the knee joint would be loaded in an undesirable manner.

The movement of the lower member with respect to the upper member upon pivoting of the lower member relative to the upper member is unequivocally fixed, as a result of which physiological movement of the knee is not allowed.

The object of the invention is to provide a knee brace by means of which an anterior-posterior shearing force can be exerted on the lower leg relative to the upper leg in a relatively simple manner whilst allowing the aforesaid physiological movement.

This object is achieved with the knee brace according to the invention and specified in claim 1.

The upper member and the lower member are each substantially independently pivotable with respect to the hinge member, and consequently they are substantially independently movable with respect to each other. Since the hinge member itself is attached to the upper leg and the shin portion by means of attachment, for example straps, the anterior-posterior shearing force is exerted on the lower leg directly from the hinge member.

EP-0.941.722 discloses a device comprising a first frame element with a shape such that it fits around the rear side of the upper leg, a second frame element with a shape such that it fits around the front side of the lower leg, and limiting means which extend from the first to the second frame element, which limiting means limit the displacement of the second frame element relative to the first frame element when the device is in position around a knee when the knee is straightened.

The knee brace according to the invention is characterised in that the hinge member comprises a first and a second hinge part, which first and second hinge parts are pivotally interconnected by a third pivot pin, wherein the first hinge part is provided with the first pivot pin at a position spaced from the third pivot pin, whilst the second hinge part is provided with the second pivot pin at a position spaced from the third pivot pin.

The first, the second and the third pivot pin enable the upper member and the lower member to pivot comparatively freely with respect to each other but also to shift with respect to each other. This aspect renders the knee brace suitable for use also with persons with individually varying knee-axis displacements upon flexion and extension. Moreover, in this way the knee brace will be able to follow the natural movement upon pivoting of the leg in an effective manner. The upper member and the lower member will remain relatively rigidly connected to the upper leg and the lower leg during said movement, and there will be hardly any undesirable relative movement between the upper member and the upper leg respectively the lower member and the lower leg.

The knee brace according to the invention is further characterised in that the second hinge part comprises a lever positioned between the second pivot pin and the third pivot pin, which lever is pivotable about a fourth pivot pin and which is connected to the means of attachment on one side and which is pivotally connected to a connecting element by a fifth pivot pin on a side remote from the means of attachment, which connecting element is pivotally connected to the first hinge part by a sixth pivot pin with an end remote from the fifth pivot pin.

Upon pivoting of the first hinge part with respect to the second hinge part from a position associated with the flexed position to a position associated with the extended position, the side of the lever connected to the means of attachment is moved in the direction of the second hinge part by pivoting about the fourth pivot pin. This leads to a defined anterior-posterior movement being imposed on the lower leg by the means of attachment connected to the shin portion.

Yet another embodiment of the knee brace according to the invention is characterised in that the angle through which the upper member can pivot with respect to the hinge is limited.

As a result, the upper member can only pivot through a preset angle with respect to the hinge, for example in an extended position. In this way the upper member and thus the upper leg are prevented from pivoting any further with respect to the hinge in a simple manner.

Yet another embodiment of the knee brace according to the invention is characterised in that the angle through which the lower member can pivot with respect to the hinge is limited.

As a result, the lower member can only pivot through a preset angle with respect to the hinge, for example in an extended position. In this way the lower member and thus the lower leg are prevented from pivoting any further with respect to the hinge in a simple manner.

Yet another embodiment of the knee brace according to the invention is characterised in that the hinge comprises an adjustable stop for setting the maximum angle to be realised between the first and the second hinge part in an extended position of the leg.

In this way it is possible to set the maximum angle to be realised between the first and the second hinge part in an extended position of the leg individually for each person in combination with the proximate and distal stops, so that the knee joint will not be overloaded.

Yet another embodiment of the knee brace according to the invention is characterised in that the hinge comprises an adjustable stop for setting the minimum angle to be realised between the first and the second hinge part in a flexed position of the leg.

In this way it is possible to set the minimum angle to be realised between the first and the second hinge part in a flexed position of the leg individually for each person in combination with the proximate and distal stops, so that the knee joint will not be overloaded.

Yet another embodiment of the knee brace according to the invention is characterised in that the adjustable stop comprises at least one disc which is rotatable about the third pivot pin, which disc can be adjustably fixed in a number of positions with respect to the first or the second hinge part, and which is provided with a stop boss, whilst the second or the first hinge part is provided with a ratchet stop that mates with said stop boss.

Such a rotatable disc makes it possible in a simple manner to realise a large number of positions and thus maximum or minimum angles to be realised between the first and the second hinge part.

Yet another embodiment of the knee brace according to the invention is characterised in that the upper member and the lower member can pivot under spring force with respect to each other upon pivoting of the leg from a flexed position to an extended position.

As a result of said spring force, the upper member and the lower member will preferably take up a position associated with the extended position of the leg.

Yet another embodiment of the knee brace according to the invention is characterised in that said spring force is obtained at least from a leaf spring that extends between the upper member and the lower member.

A leaf spring makes it possible in a simple manner to obtain a spring force. Moreover, a leaf spring allows both relative pivoting movement and relative translating movement of the lower member with respect to the upper member. Movements and rotation in the frontal plane are prevented in a simple manner by the use of a leaf spring.

The invention will now be explained in more detail with reference to the drawings, in which:
Figure 1 is a side view of a part of the knee brace according to the invention in different positions thereof;
Figure 2 is a perspective, exploded view of a part of the knee brace shown in figure 1;
Figure 3 is a perspective, exploded view of a part of the knee brace shown in figure 2;
Figure 4 is a perspective view of a part of the knee brace shown in figure 2;
Figure 5 is a perspective, exploded view of the part of a knee brace according to the invention that is shown in figure 4.

Like parts are indicated by the same numerals in figures.

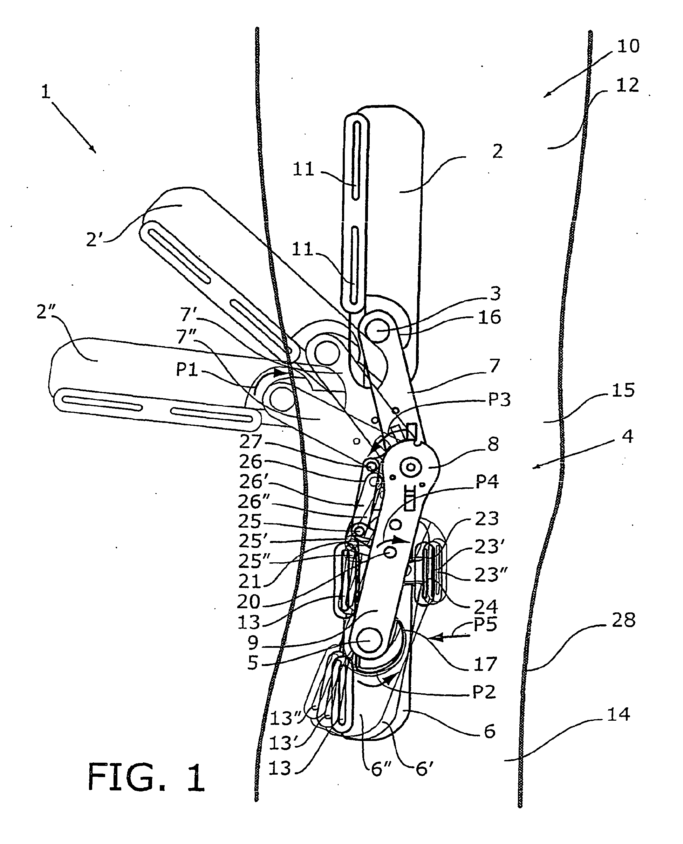

Figure 1 is a side view of a knee brace 1 according to the invention in different positions thereof. The knee brace 1 comprises an upper member 2, a hinge member 4, which is pivotally connected to the upper member 2 by a first pivot pin 3, and a lower member 6, which is pivotally connected to the hinge member 4 by a second pivot pin 5. The hinge member 4 comprises a first hinge part 7, which is pivotally connected to the upper member 2 by the first pivot pin 3. The first hinge part 7 is pivotally connected to a second hinge part 9 of the hinge member 4 by a third pivot pin 8. The second hinge part 9 is pivotally connected to the lower member 6 by the second pivot pin 5.

The knee brace 1 preferably comprises two assemblies, each comprising an upper member 2, a hinge member 4, and a lower member 6, and such an assembly is positioned on either side of a leg 10 of a person. Each upper member 2 is provided with two slots 11. The knee brace 1 is provided with tightenable straps which are known per se, which straps are passed through the slots 11 of the two upper members 2 for attaching the upper member 2 to an upper leg portion 12 of the leg 10.

In a similar fashion, the lower member 6 is provided with two slots 13, through which straps are passed for attaching the lower members 6 to a lower leg portion 14 of the leg 10. Positioned between the upper leg portion 12 and the lower leg portion 14 is the knee portion 15 of the leg 10.

The upper member 2 is provided with a stop 16 for limiting the angle through which the upper member 2 can pivot in the direction indicated by the arrow P1 with respect to the first hinge part 7. In a similar fashion, the lower member 6 is provided with a stop 17 for limiting the angle through which the lower member 6 can pivot in the direction indicated by the arrow P2 with respect to the second hinge part 9.

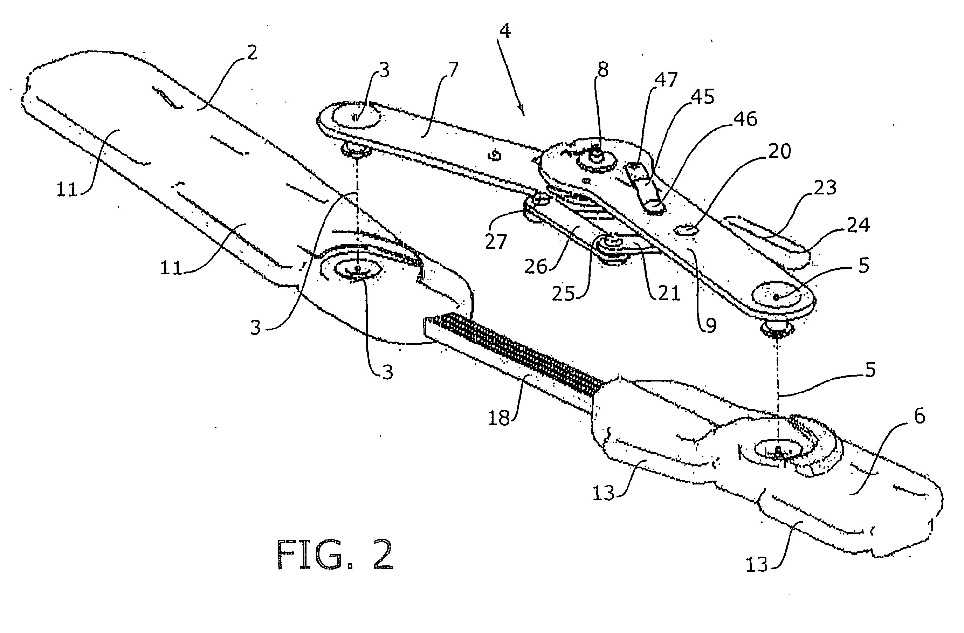

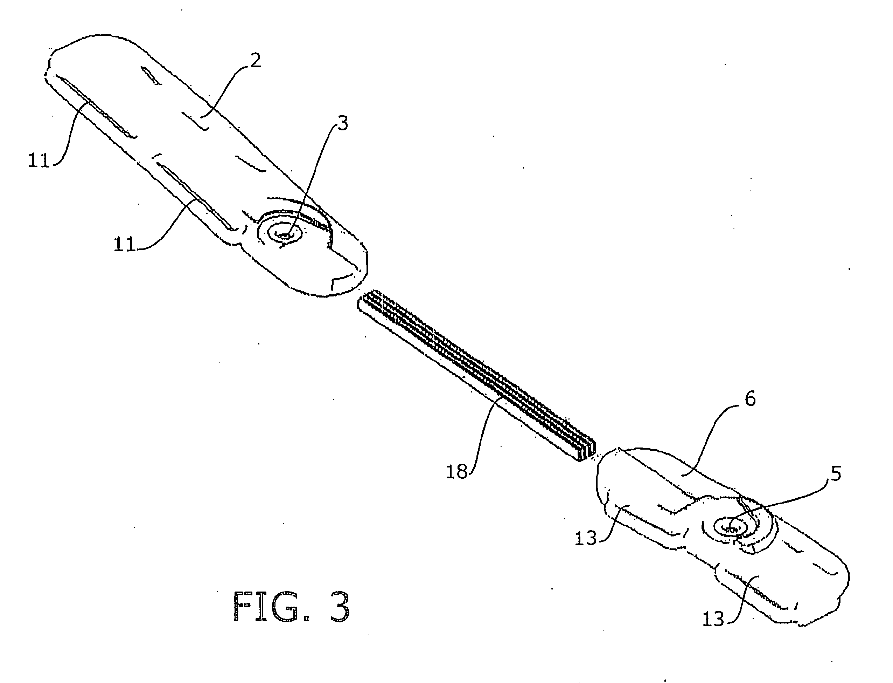

As is clearly shown in figure 2 and figure 3, the upper member 2 and the lower member 6 are interconnected by a number of leaf springs 18 extending parallel to each other. Said leaf springs 18 are not shown in figure 1 for the sake of clarity of the drawing of figure 1. Because of the presence of the leaf springs 18, the upper member 2 and the lower member 6 can pivot about the pivot pins 3, 8, 5 with respect to each other and be moved towards each other against the spring force of the leaf springs 18. However, the leaf springs 18 also provide a certain degree of rigidity against pivoting of the upper member 2 with respect to the lower member 6 about axes extending transversely to the pins 3, 8, 5.

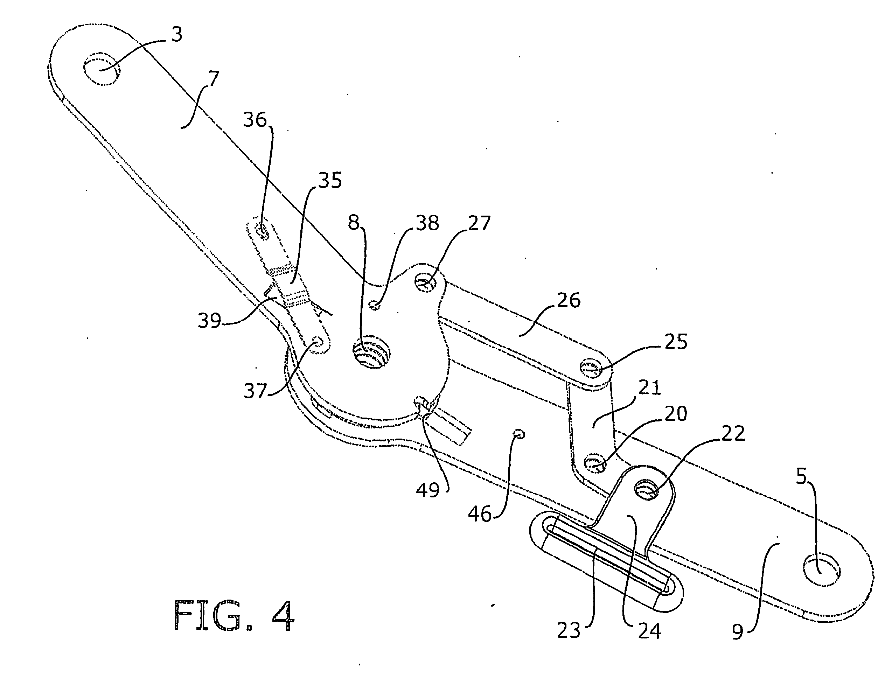

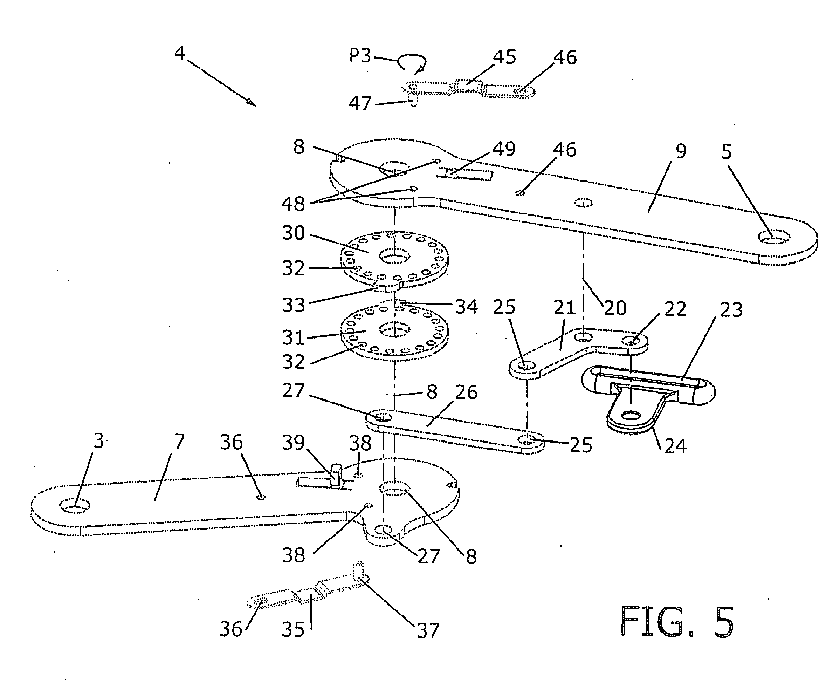

The construction of the hinge 4 will now be explained in more detail with reference to figures 1-5. The hinge 4 comprises a lever 21, which is pivotally connected to the second hinge part 9 by a fourth pivot pin 20. Said fourth pivot pin 20 is located between the second pivot pin 5 and the third pivot pin 8. The lever 21 is provided at a first end 22 thereof with a means of attachment 24 comprising a slot 23. The means of attachment 24 pivots about the end 22.

On a side remote from the end 22, the lever 21 is pivotally connected by a fifth pivot pin 25 to a connecting rod 26 forming a connecting element, which connecting rod is pivotally connected on a side remote from the fifth pivot pin 25 to the first hinge part 7 by a sixth pivot pin 27. The means of attachment 24 are connected to the shin portion 28 of the leg 10 by means of straps, which are passed through the slots 23 positioned on either side of the leg 10.

The hinge 4 further comprises two discs 30, 31 positioned between the first hinge part and 7 and the second hinge part 9, which discs are rotatable about the pivot pin 8. Each disc 30, 31 is circumferentially provided with a number of regularly spaced-apart openings 32 and a projecting cam 33, 34.

The first hinge part 7 is provided with an adjusting arm 35, which is pivotally connected to the first hinge part by a pivot pin 36 and which is provided with a locking pin 37 on a side remote from the pivot pin 36, which locking pin, depending on the position of the adjusting arm 35, extends through one of the passages 38 in the first hinge part 7. The pin 37 further extends through one of the openings 32 in the disc 31. In this way the position of the stop 34 with respect to the first hinge part 7 is adjustable. Depending on the passage 38 that is selected, other positions can be selected. The angle between the passages 38 and the pivot pin 8 is not a multiple of the angle between the openings 32 and the pivot pin 8.

The first hinge part 7 is further provided with a stop 39, which has been formed by cutting out and bending part of the plate material from which the first hinge part 7 is made.

The second hinge part 9 is provided with an adjusting arm 45 in a similar manner as the first hinge part 7, which adjusting arm is pivotable about a pivot pin 46 and which is provided with a locking pin 47. The locking pin 47 is positionable through one of the passages 48 so as to be brought into engagement with one of the openings 32 in the disc 30. In this way the disc 32, and consequently the cam 33, is adjustable with respect to the second hinge part 9. The second hinge part 9 is provided with a stop 49.

Upon pivoting of the first hinge part 7 with respect to the second hinge part 9 in the direction indicated by the arrow P3, the stop 39 will come into contact with the cam 33, so that a further increase of the angle between the first hinge part 7 and the second hinge part 9 is prevented in a simple manner.

In a similar fashion, the minimum angle to be realised between the first hinge part 7 and the second hinge part 9 will be obtained as soon as the stop 49 comes into contact with the cam 34 upon pivoting of the first hinge part 7 with respect to the second hinge part 9 in a direction opposite the direction indicated by the arrow P3.

Both the maximum angle and the minimum angle to be realised between the first hinge part 7 and the second hinge part 9 can be set in a simple manner by adjusting the discs 30, 31 and inserting the locking pins 37, 47 into the openings 32 in the discs 30, 31 in question.

The operation of the knee brace 1 will now be explained in more detail with reference to figures 1-5.

Before the knee brace 1 is put on, the discs 30, 31 are placed in the desired positions and the adjusting arms 35, 45 are connected to the respective hinge parts 7, 9.

As already indicated above, the upper members 2 of the knee brace positioned on either side of the leg 2 are attached to the upper leg 12 by means of straps extending through the slots 11.

Similarly, the lower members 6 are attached to the lower leg 14 by means of straps extending through the slots 13.

The levers 21 extending on either side of the leg 10 are interconnected by straps extending through the slots 23 of the means of attachment 24, which straps abut against the shin portion 28 of the lower leg 14.

In figure 1, different positions of one and the same part are indicated ' and ". For the sake of clarity, the second hinge part 9 is shown in the same position for all three positions that are shown. In a flexed position of the leg 10, the upper member 2" includes the minimum angle to be realised with the lower member 6". Upon extension of the leg 10, the upper member 2" is pivoted in the direction indicated by the arrow P1 about the first pivot pin 3, until the stop 16 abuts against the first hinge part 7. Simultaneously with the pivoting of the upper member 2" to the position indicated at 2, via the position indicated at 2', the first hinge part 7" is pivoted about the third pivot pin 8 with respect to the second hinge part 9 to the position indicated at 7, via the position indicated at 7", until the stop 34 abuts against the cam 49.

Partially under the influence of the spring force generated by the leaf spring 18, the lower member 6" will pivot in the direction indicated by the arrow P2 about the second pivot pin 5 until the stop 17 abuts against the second hinge part 9. The lower member 6" will successively take up the positions indicated at 6' and 6.

Upon pivoting of the first hinge part 7 in the direction indicated by the arrow P3, the connecting rod 26 will pivot about the pivot pin 27, causing the pivot pin 25 connected to the lever 21 to pivot about the fourth pivot pin 20 in the direction indicated by the arrow P4, as a result of which also the end 22 connected to the lever 21 will be pivoted in the direction indicated by the arrow P4. The means of attachment 24 is pivotally connected to the end 22, so that the means of attachment 24 will be moved in the direction indicated by the arrow P5 towards the second hinge part 7 upon pivoting of the lever 21 about the fourth pivot pin 20.

During said movement, the strap connected to the means of attachment 24 will exert an anterior-posterior shearing force on the shin portion 28 of the lower leg 14. In a sound leg 10, such a force is generated by cruciate ligaments in the knee 4. In a damaged knee, the anterior cruciate ligament no longer functions and said force is now generated by the knee brace 1 according to the invention.

## Claims

1. A knee brace (1) which can be detachably attached to a leg (10) comprising an upper leg portion (12), a knee portion (15) and a lower leg portion (14) comprising a shin portion (28), which leg (10) can pivot from an extended position to a flexed position and vice versa, which knee brace (1) at least comprises at least one upper member (2) to be attached to the upper leg portion (12), at least one lower member (6) to be connected to the lower leg portion (14), at least one hinge member (4), which is pivotally connected to the upper member (2) by a first pivot pin (3) and which is pivotally connected to the lower member (6) by a second pivot pin (5), and means for exerting an anterior-posterior shearing force on the lower leg portion (14) relative to the upper leg portion (12) upon pivoting of the leg (10) from the flexed position to the extended position, **characterised In that** said means comprise means of attachment (24) connected to the hinge member (4) for attaching the hinge member (4) to the shin portion (28), which means of attachment (24) are movable in anterior-posterior direction with respect to the hinge member (4) upon pivoting of the leg (10) from the flexed position to the extended position, wherein the hinge member (4) comprises a first and a second hinge part (7, 9), which first and second hinge parts (7, 9) are pivotally interconnected by a third pivot pin (8), wherein the first hinge part (7) is provided with the first pivot pin (3) at a position spaced from the third pivot pin (8), whilst the second hinge part (9) Is provided with the second pivot pin (5) at a position spaced from the third pivot pin (8), wherein the second hinge part (9) comprises a lever (21) positioned between the second pivot pin (5) and the third pivot pin (8), which lever (21) is pivotable about a fourth pivot pin (20) and which is connected to the means of attachment (24) on one side and which is pivotally connected to a connecting element (26) by a fifth pivot pin (25) on a side remote from the means of attachment (24), which connecting element is pivotally connected to the first hinge part (7) by a sixth pivot pin (27) with an end remote from the fifth pivot pin (25).

2. A knee brace (1) according to any one of the preceding claims, **characterised In that** the angle through which the upper member (2) can pivot with respect to the hinge member (4) is limited.

3. A knee brace (1) according to any one of the preceding claims, **characterised in that** the angle through which the lower member (6) can pivot with respect to the hinge member (4) is limited.

4. A knee brace (1) according to any one of the preceding claims, **characterised in that** the hinge member (4) comprises an adjustable stop (16) for setting the maximum angle to be realised between the first and the second hinge part (7, 9) in an extended position of the leg (10).

5. A knee brace (1) according to any one of the preceding claims, **characterised In that** the hinge member (4) comprises an adjustable stop (16) for setting the minimum angle to be realised between the first and the second hinge part (7, 9) In a flexed position of the leg (10).

6. A knee brace (1) according to any one of the preceding claims, **characterised In that** the adjustable stop (16) comprises at least one disc (30) which is rotatable about the third pivot pin (8), which disc (30) can be adjustably fixed in a number of positions with respect to the first or the second hinge part (7, 9), and which is provided with a stop boss, whilst the second or the first hinge part (9, 7) is provided with a ratchet stop that mates with said stop boss.

7. A knee brace (1) according to any one of the preceding claims, **characterised in that** the upper member (2) and the lower member (6) can pivot under spring force with respect to each other upon pivoting of the leg (10) from a flexed position to an extended position.

8. A knee brace (1) according to claim 7, **characterised In that** said spring force Is obtained at least from a leaf spring that extends between the upper member (2) and the lower member (8).

9. A hinge member (4) suitable for use In the knee brace (1) according to any one of the proceeding claims, **characterised in that** it comprises means of attachment (24) which are connected to the hinge member (4) for attaching the hinge member (4) to a shin portion (28) of a lower leg portion (14) of a leg (10), which means of attachment (24) are movable in anterior-posterior direction with respect to the hinge member (4) upon pivoting of the leg (10) from a flexed position to an extended position, wherein the hinge member (4) comprises a first and a second hinge part (7, 9), which first and second hinge parts (7, 9) are pivotally Interconnected by a third pivot pin (8), wherein the first hinge part (7) is provided with a first pivot pin (3) at a position spaced from the third pivot pin (8), whilst the second hinge part (9) Is provided with a second pivot pin (5) at a position spaced from the third pivot pin (8), wherein the
second hinge part (9) comprises a lever (21) positioned between the second pivot
pin (5) and the third pivot pin (8), which lever (21) is pivotable about a fourth pivot pin (20) and which is connected to the means of attachment (24) on one side and which is pivotally connected to a connecting element (26) by a fifth pivot pin (25) on a side remote from the means of attachment (24), which connecting element is pivotally connected to the first hinge part (7) by a sixth pivot pin (27) with an end remote from the fifth pivot pin (25).

## Patentansprüche

1. Kniestütze (1), die abnehmbar an einem Bein (10) befestigt werden kann, das einen oberen Beinbereich (12), einen Kniebereich (15) und einen unteren Beinbereich (14) umfasst, der einen Schienbeinbereich (28) aufweist, wobei das Bein (10) aus einer gestreckten Position in eine gebeugte Position geschwenkt werden kann und umgekehrt, wobei die Kniestütze (1) zumindest mindestens ein oberes Element (2), das am oberen Beinbereich (12) zu befestigen ist, mindestens ein unteres Element (6), das mit dem unteren Beinbereich (14) zu verbinden ist, mindestens ein Gelenkelement (4), das über einen ersten Schwenkzapfen (3) schwenkbar mit dem oberen Element (2) verbunden ist und über einen zweiten Schwenkzapfen (5) schwenkbar mit dem unteren Element (6) verbunden ist, und Einrichtungen aufweist, um beim Verschwenken des Beins (10) aus der gebeugten Position in die gestreckte Position eine nach vorne bzw. hinten wirkende Scherkraft auf den unteren Beinbereich (14) relativ zum oberen Beinbereich (12) auszuüben, **dadurch gekennzeichnet, dass** die Einrichtungen Befestigungseinrichtungen (24) umfassen, die mit dem Gelenkelement (4) verbunden sind, um das Gelenkelement (4) am Schienbeinbereich (28) zu befestigen, wobei die Befestigungseinrichtungen (24) beim Verschwenken des Beins (10) aus der gebeugten Position in die gestreckte Position in eine in Bezug auf das Gelenkelement (4) nach vorne bzw. hinten führende Richtung bewegbar sind, wobei das Gelenkelement (4) ein erstes und ein zweites Gelenkteil (7, 9) aufweist, wobei das erste und zweite Gelenkteil (7, 9) durch einen dritten Schwenkzapfen (8) schwenkbar miteinander verbunden sind, wobei das erste Gelenkteil (7) an einer vom dritten Schwenkzapfen (8) beabstandeten Position mit dem ersten Schwenkzapfen (3) versehen ist, während das zweite Gelenkteil (9) an einer vom dritten Schwenkzapfen (8) beabstandeten Position mit dem zweiten Schwenkzapfen (5) versehen ist, wobei das zweite Gelenkteil (9) einen Hebel (21) aufweist, der zwischen dem zweiten Schwenkzapfen (5) und dem dritten Schwenkzapfen (8) angeordnet ist, welcher Hebel (21) um einen vierten Schwenkzapfen (20) schwenkbar ist und an einer Seite mit den Befestigungseinrichtungen (24) verbunden ist und an einer von den Befestigungseinrichtungen (24) entfernten Seite über einen fünften Schwenkzapfen (25) schwenkbar mit einem Verbindungselement (26) verbunden ist, welches Verbindungselement mit einem vom fünften Schwenkzapfen (25) entfernten Ende über einen sechsten Schwenkzapfen (27) schwenkbar mit dem ersten Gelenkteil (7) verbunden ist.

2. Kniestütze (1) nach einem der vorhergehenden Ansprüche, **dadurch gekennzeichnet, dass** der Winkel, über den das obere Element (2) in Bezug auf das Gelenkelement (4) verschwenken kann, begrenzt ist.

3. Kniestütze (1) nach einem der vorhergehenden Ansprüche, **dadurch gekennzeichnet, dass** der Winkel, über den das untere Element (6) in Bezug auf das Gelenkelement (4) verschwenken kann, begrenzt ist.

4. Kniestütze (1) nach einem der vorhergehenden Ansprüche, **dadurch gekennzeichnet, dass** das Gelenkelement (4) einen verstellbaren Anschlag (16) umfasst, um den Maximalwinkel einzustellen, der in einer gestreckten Position des Beins (10) zwischen dem ersten und zweiten Gelenkteil (7, 9) zu erzielen ist.

5. Kniestütze (1) nach einem der vorhergehenden Ansprüche, **dadurch gekennzeichnet, dass** das Gelenkelement (4) einen verstellbaren Anschlag (16) umfasst, um den Minimalwinkel einzustellen, der in einer gebeugten Position des Beins (10) zwischen dem ersten und zweiten Gelenkteil (7, 9) zu erzielen ist.

6. Kniestütze (1) nach einem der vorhergehenden Ansprüche, **dadurch gekennzeichnet, dass** der einstellbare Anschlag (16) mindestens eine Scheibe (30) aufweist, die um den dritten Schwenkzapfen (8) drehbar ist, wobei die Scheibe (30) in einer Anzahl von Positionen in Bezug auf das erste oder zweite Gelenkteil (7, 9) einstellbar fixiert werden kann und mit einem Haltevorsprung versehen ist, während das zweite oder erste Gelenkteil (9, 7) mit einem Ratschenanschlag versehen ist, der mit dem Haltevorsprung zusammenpasst.

7. Kniestütze (1) nach einem der vorhergehenden Ansprüche, **dadurch gekennzeichnet, dass** beim Verschwenken des Beins (10) aus einer gebeugten Position in eine gestreckte Position das obere Element (2) und das untere Element (6) unter Federkraft in Bezug aufeinander verschwenken können.

8. Kniestütze (1) nach Anspruch 7, **dadurch gekennzeichnet, dass** die Federkraft von zumindest einer Blattfeder erhalten wird, die sich zwischen dem oberen Element (2) und dem unteren Element (6) erstreckt.

9. Gelenkelement (4), das sich zur Verwendung in der Kniestütze (1) nach einem der vorhergehenden Ansprüche eignet, **dadurch gekennzeichnet, dass** es Befestigungseinrichtungen (24) umfasst, die mit dem Gelenkelement (4) verbunden sind, um das Gelenkelement (4) an einem Schienbeinbereich (28) eines unteren Beinbereichs (14) eines Beins (10) zu befestigen, wobei die Befestigungseinrichtungen (24) beim Verschwenken des Beins (10) aus einer gebeugten Position in eine gestreckte Position in eine in Bezug auf das Gelenkelement (4) nach vorne bzw. hinten führende Richtung bewegbar sind, wobei das Gelenkelement (4) ein erstes und ein zweites Gelenkteil (7, 9) aufweist, wobei das erste und zweite Gelenkteil (7, 9) durch einen dritten Schwenkzapfen (8) schwenkbar miteinander verbunden sind, wobei das erste Gelenkteil (7) an einer vom dritten Schwenkzapfen (8) beabstandeten Position mit einem ersten Schwenkzapfen (3) versehen ist, während das zweite Gelenkteil (9) an einer vom dritten Schwenkzapfen (8) beabstandeten Position mit einem zweiten Schwenkzapfen (5) versehen ist, wobei das zweite Gelenkteil (9) einen Hebel (21) aufweist, der zwischen dem zweiten Schwenkzapfen (5) und dem dritten Schwenkzapfen (8) angeordnet ist, welcher Hebel (21) um einen vierten Schwenkzapfen (20) schwenkbar ist und an einer Seite mit den Befestigungseinrichtungen (24) verbunden ist und an einer von den Befestigungseinrichtungen (24) entfernten Seite über einen fünften Schwenkzapfen (25) schwenkbar mit einem Verbindungselement (26) verbunden ist, welches Verbindungselement mit einem vom fünften Schwenkzapfen (25) entfernten Ende über einen sechsten Schwenkzapfen (27) schwenkbar mit dem ersten Gelenkteil (7) verbunden ist.

## Revendications

1. Orthèse de genou (1), pouvant être fixée de manière détachable à une jambe (10) comprenant une partie de jambe supérieure (12), une partie de genou (15) et une partie de jambe inférieure (14) comprenant une partie de jambe de devant (28), ladite jambe (10) pouvant pivoter depuis une position étendue vers une position fléchie et inversement, ladite orthèse de genou (1) comprenant au moins un élément supérieur (2) à fixer à la partie de jambe supérieure (12), au moins un élément inférieur (6) à raccorder à la partie de jambe inférieure (14), au moins un élément charnière (4), qui est raccordé de manière pivotante à l'élément supérieur (2) par une première broche de pivot (3) et qui est raccordé de manière pivotante à l'élément inférieur (6) par une seconde broche de pivot (5), et des moyens pour exercer une force de cisaillement antérieure-postérieure sur la partie de jambe inférieure (14) relativement à la partie de jambe supérieure (12), lors du pivotement de la jambe (10) de la position fléchie à la position étendue, **caractérisée en ce que** lesdits moyens comprennent des moyens de fixation (24) raccordés à l'élément charnière (4), afin de fixer l'élément charnière (4) à la partie de jambe de devant (28), lesdits moyens de fixation (24) étant mobiles dans une direction antérieure-postérieure relativement à l'élément charnière (4) lors du pivotement de la jambe (10) de la position fléchie à la position étendue, dans laquelle l'élément charnière (4) comprend une première et une seconde parties charnières (7, 9), lesdites première et seconde parties charnières (7, 9) étant interconnectées de manière pivotante par une troisième broche de pivot (8), dans laquelle la première partie charnière (7) est dotée de la première broche de pivot (3) dans une position espacée par rapport à la troisième broche de pivot (8), tandis que la seconde partie charnière (9) est dotée de la seconde
broche de pivot (5) dans une position espacée par rapport à la troisième broche de pivot (8), dans laquelle la seconde partie charnière (9) comprend un levier (21) positionné entre la seconde broche de pivot (5) et la troisième broche de pivot (8), ledit levier (21) pouvant pivoter autour d'une quatrième broche de pivot (20) et étant raccordé aux moyens de fixation (24) sur un côté et étant raccordé de manière pivotante à un élément de connexion (26) par une cinquième broche de pivot (25) sur un côté éloigné des moyens de fixation (24), ledit élément de connexion étant raccordé de manière pivotante à la première partie charnière (7) par une sixième broche de pivot (27) avec une extrémité éloignée de la cinquième broche de pivot (25).

2. Orthèse de genou (1) selon l'une quelconque des revendications précédentes, **caractérisée en ce que** l'angle à travers lequel l'élément supérieur (2) peut pivoter relativement à l'élément charnière (4) est limité.

3. Orthèse de genou (1) selon l'une quelconque des revendications précédentes, **caractérisée en ce que** l'angle à travers lequel l'élément inférieur (6) peut pivoter relativement à l'élément charnière (4) est limité.

4. Orthèse de genou (1) selon l'une quelconque des revendications précédentes, **caractérisée en ce que** l'élément charnière (4) comprend une butée réglable (16) afin de régler l'angle maximum à réaliser entre la première et la seconde parties charnières (7, 9) dans une position étendue de la jambe (10).

5. Orthèse de genou (1) selon l'une quelconque des revendications précédentes, **caractérisée en ce que** l'élément charnière (4) comprend une butée réglable (16) afin de régler l'angle minimum à réaliser entre la première et la seconde parties charnières (7, 9) dans une position fléchie de la jambe (10).

6. Orthèse de genou (1) selon l'une quelconque des revendications précédentes, **caractérisée en ce que** la butée réglable (16) comprend au moins un disque (30) qui peut tourner autour de la troisième broche de pivot (8), ledit disque (30) pouvant être fixé de manière ajustable dans un certain nombre de positions relativement à la première ou à la seconde parties charnières (7, 9) et qui est doté d'un bossage de butée, tandis que la seconde ou la première parties
charnières (9, 7) sont dotées d'une butée à cliquet qui s'accouple audit bossage de butée.

7. Orthèse de genou (1) selon l'une quelconque des revendications précédentes, **caractérisée en ce que** l'élément supérieur (2) et l'élément inférieur (6) peuvent pivoter sous la force d'un ressort, l'un par rapport à l'autre, lors du pivotement de la jambe (10) d'une position fléchie à une position étendue.

8. Orthèse de genou (1) selon la revendication 7, **caractérisée en ce que** ladite force de ressort est obtenue au moins à partir d'un ressort à lames, qui s'étend entre l'élément supérieur (2) et l'élément inférieur (6).

9. Élément charnière (4) adapté pour une utilisation dans l'orthèse de genou (1) selon l'une quelconque des revendications précédentes, **caractérisé en ce qu'**il comprend des moyens de fixation (24) qui sont raccordés à l'élément charnière (4) afin de fixer
l'élément charnière (4) à une partie de jambe de devant (28) d'une partie de jambe inférieure (14) d'une jambe (10), lesdits moyens de fixation (24) étant mobiles dans la direction antérieure-postérieure relativement à l'élément charnière (4) lors du pivotement de la jambe (10) d'une position fléchie à une position étendue, dans lequel l'élément charnière (4) comprend une première et une seconde parties charnières (7, 9), lesdites première et seconde parties charnières (7, 9) étant interconnectées de manière pivotante par une troisième broche de pivot (8), dans lequel la première partie charnière (7) est dotée d'une première broche de pivot (3) dans une position espacée par rapport à la troisième broche de pivot (8),
tandis que la seconde partie charnière (9) est dotée d'une seconde broche de pivot (5), dans une position espacée par rapport à la troisième broche de pivot (8), dans lequel la seconde partie charnière (9) comprend un levier (21) positionné entre la seconde broche de pivot (5) et la troisième broche de pivot (8), ledit levier (21) pouvant pivoter autour d'une quatrième broche de pivot (20) et étant raccordé aux moyens de fixation (24) sur un côté et étant raccordé de manière pivotante à un élément de connexion (26) par une cinquième broche de pivot (25) sur un côté éloigné desdits moyens de fixation (24), ledit élément de connexion étant raccordé de manière pivotante à la première partie charnière (7) par une sixième broche de pivot (27) avec une extrémité éloignée de la cinquième broche de pivot (25).
